# FASCICULE DE BREVET EUROPEEN

(11) **EP 2 807 247 B1**
(45) Date de publication et mention de la délivrance du brevet: **25.10.2017**
(21) Numéro de dépôt: 13710560.7
(22) Date de dépôt: 24.01.2013
(51) Int. Cl.: C12N 1/19, C12N 9/10, C07K 14/395, C12Q 1/68, C12G 1/00, C12P 3/00, C12P 7/24

(54) **MÉTHODE DE CONTRÔLE DE LA PRODUCTION DE SULFITES, D'HYDROGÈNE SULFUREUX ET D'ACÉTALDÉHYDE PAR DES LEVURES**
VERFAHREN ZUR STEUERUNG DER HERSTELLUNG VON SULFITEN, SCHWEFELWASSERSTOFF UND ACETALDEHYD DURCH HEFEN
METHOD FOR CONTROLLING THE PRODUCTION OF SULPHITES, OF HYDROGEN SULPHIDE AND OF ACETALDEHYDE BY YEASTS

(30) Priorité: 25.01.2012 FR 1250717
(43) Date de publication de la demande: 03.12.2014
(73) Titulaire: Institut National De La Recherche Agronomique, 75007 Paris (FR); Centre international d'études supérieures en sciences agronomiques Montpellier Supagro, 34060 Montpellier cedex 02 (FR)
(72) Inventeur: BLONDIN, Bruno, 34070 Montpellier (FR); NOBLE, Jessica, 34970 Lattes (FR); SANCHEZ, Isabelle, 34090 Montpellier (FR)
(74) Mandataire: Gevers & Orès
(86) Numéro de dépôt international: PCT/IB2013/050623
(87) Numéro de publication internationale: WO 2013/111091

(56) Documents cités:
- WO-A2-2008/115759
- SATOSHI YOSHIDA ET AL: "A novel mechanism regulates H2S and SO2 production in Saccharomyces cerevisiae", YEAST, vol. 28, no. 2, 1 février 2011 (2011-02-01), pages 109-121, XP055037473, ISSN: 0749-503X, DOI: 10.1002/yea.1823
- HANSEN J ET AL: "Inactivation of MET2 in brewer's yeast increases the level of sulfite in beer", JOURNAL OF BIOTECHNOLOGY, ELSEVIER SCIENCE PUBLISHERS, AMSTERDAM, NL, vol. 50, no. 1, 13 septembre 1996 (1996-09-13), pages 75-87, XP004037068, ISSN: 0168-1656, DOI: 10.1016/0168-1656(96)01551-9 cité dans la demande
- HYUN SEOK KIM ET AL: "Dissecting the pleiotropic consequences of a quantitative trait nucleotide", FEMS YEAST RESEARCH, vol. 9, no. 5, 1 août 2009 (2009-08-01), pages 713-722, XP055037475, ISSN: 1567-1356, DOI: 10.1111/j.1567-1364.2009.00516.x
- Berlese-Noble Jessica et al.: "A new and powerful strategy to control SO2 and H2S production by wine yeasts", ICY2012, Poster Session Food and Beverage (Area H) , 28 août 2012 (2012-08-28), XP002683336, Extrait de l'Internet: URL:http://conferencing.uwex.edu/conferenc es/icy2012/documents/AreaHPosters_000.pdf [extrait le 2012-09-12]

### SEQUENCE LISTING

<110> INSTITUT NATIONAL DE LA RECHERCHE AGRONOMIQUE
   CENTRE INTERNATIONAL D'ETUDES SUPERIEURES EN SCIENCES
   AGRONOMIQUES (MONTPELLIER SUPAGRO)
   BLONDIN, Bruno
   NOBLE, Jessica
   SANCHEZ, Isabelle
<120> MÉTHODE DE CONTRÔLE DE LA PRODUCTION DE SULFITES, D'HYDROGÈNE SULFUREUX ET D'ACÉTALDÉHYDE PAR DES LEVURES
<130> MJP-11-F539-154PCT
<150> FR1250717
   <151> 2012-01-25
<160> 6
<170> PatentIn version 3.5
<210> 1
   <211> 2292
   <212> DNA
   <213> Saccharomyces cerevisiae
<220>
   <221> CDS
   <222> (1)..(2292)
<400> 1
   atg aaa cgg ttg caa ttg ttt ggt aga tca aaa tat ttc tcg ctg gtc          48
   Met Lys Arg Leu Gln Leu Phe Gly Arg Ser Lys Tyr Phe Ser Leu Val
   1               5                   10                  15
   tcc agt gct gcg aag gag gaa gaa gaa gag gaa gag ggc tgt gct gat          96
   Ser Ser Ala Ala Lys Glu Glu Glu Glu Glu Glu Glu Gly Cys Ala Asp
               20                  25                  30
   gcc aaa agc ctt cta cat agt acg agc cat gat atc aaa tcg aga tca         144
   Ala Lys Ser Leu Leu His Ser Thr Ser His Asp Ile Lys Ser Arg Ser
           35                  40                  45
   cta cgc ttc aac gat aaa tcg tcc ctc atg tgt ttg cca aca aaa gtt         192
   Leu Arg Phe Asn Asp Lys Ser Ser Leu Met Cys Leu Pro Thr Lys Val
       50                  55                  60
   cta ctg tta ata ctg cga act ttg gac ttc aat aca tta gta aca cta         240
   Leu Leu Leu Ile Leu Arg Thr Leu Asp Phe Asn Thr Leu Val Thr Leu
   65                  70                  75                  80
   tgc caa gtc aat tcg agg ttc tac aat ttg att acg aat gag ttc ctt         288
   Cys Gln Val Asn Ser Arg Phe Tyr Asn Leu Ile Thr Asn Glu Phe Leu
                   85                  90                  95
   ttc caa aac gtt att ttg gac tca aaa ctt tcg ttg tta aag ttc aat         336
   Phe Gln Asn Val Ile Leu Asp Ser Lys Leu Ser Leu Leu Lys Phe Asn
               100                 105                 110
   gct ttg ata cac tcc gag ttc cac acg tcg aac att gtc aca cac agc         384
   Ala Leu Ile His Ser Glu Phe His Thr Ser Asn Ile Val Thr His Ser
           115                 120                 125
   ggt gat tgt agc aca caa tct aga tca caa aat gca agg ttc ctc gta       432
   Gly Asp Cys Ser Thr Gln Ser Arg Ser Gln Asn Ala Arg Phe Leu Val
       130                 135                 140
   aga tcc atc gaa ttc aaa aat cct cag tcc caa gac tcg ttg tta aaa       480
   Arg Ser Ile Glu Phe Lys Asn Pro Gln Ser Gln Asp Ser Leu Leu Lys
   145                 150                 155                 160
   tac agt aag ttc tat aat aag agt ggc caa gat tct att att gct gga       528
   Tyr Ser Lys Phe Tyr Asn Lys Ser Gly Gln Asp Ser Ile Ile Ala Gly
                   165                 170                 175
   tct tat aaa ctc gat tcg tat gat aaa gac gta aaa aaa ctg aat aac       576
   Ser Tyr Lys Leu Asp Ser Tyr Asp Lys Asp Val Lys Lys Leu Asn Asn
               180                 185                 190
   atc aga cta aac gat gag act ccc atc ata act tca gaa cga atc aaa       624
   Ile Arg Leu Asn Asp Glu Thr Pro Ile Ile Thr Ser Glu Arg Ile Lys
           195                 200                 205
   ctg ctt gat aaa ttg gaa agc aac tac ttc cat tat act tat att gag       672
   Leu Leu Asp Lys Leu Glu Ser Asn Tyr Phe His Tyr Thr Tyr Ile Glu
       210                 215                 220
   cta atg ctt gat att ata gac tat cta ccg aac ctg acc aga gtg att       720
   Leu Met Leu Asp Ile Ile Asp Tyr Leu Pro Asn Leu Thr Arg Val Ile
   225                 230                 235                 240
   ctg agt gat gtg gaa ccg aat ttt aaa att cct tta tgg tac tct gta       768
   Leu Ser Asp Val Glu Pro Asn Phe Lys Ile Pro Leu Trp Tyr Ser Val
                   245                 250                 255
   ttc aac gat gga tct aga gat ttt ttc aag aaa ata atc aag ggc cag       816
   Phe Asn Asp Gly Ser Arg Asp Phe Phe Lys Lys Ile Ile Lys Gly Gln
               260                 265                 270
   caa tcc atc aca aac gaa gat ttg agg act ttc caa cta tcc aaa aaa       864
   Gln Ser Ile Thr Asn Glu Asp Leu Arg Thr Phe Gln Leu Ser Lys Lys
           275                 280                 285
   ttt gtc aaa gaa tac gaa tcc aag tat tat tct ctg cca agg ttg aaa       912
   Phe Val Lys Glu Tyr Glu Ser Lys Tyr Tyr Ser Leu Pro Arg Leu Lys
       290                 295                 300
   ata ctg gaa atc aaa gct aat aat aaa aga caa aga acc ttt aat cgt       960
   Ile Leu Glu Ile Lys Ala Asn Asn Lys Arg Gln Arg Thr Phe Asn Arg
   305                 310                 315                 320
   caa cgc cat cac caa aag ctc gta cta aga ccg agt tta ttc tgc tgt      1008
   Gln Arg His His Gln Lys Leu Val Leu Arg Pro Ser Leu Phe Cys Cys
                   325                 330                 335
   ttc ggc ata att aat gaa ctt aaa cta gaa aat gta acg ata gac acc      1056
   Phe Gly Ile Ile Asn Glu Leu Lys Leu Glu Asn Val Thr Ile Asp Thr
               340                 345                 350
   gaa tcg cta gat att cca atg gaa ttc tta cca cta ttt cta aag aac      1104
   Glu Ser Leu Asp Ile Pro Met Glu Phe Leu Pro Leu Phe Leu Lys Asn
           355                 360                 365
   gaa gat aat gaa ctg tac agt tta cag tct cct atc act gca ctt act      1152
   Glu Asp Asn Glu Leu Tyr Ser Leu Gln Ser Pro Ile Thr Ala Leu Thr
           370                 375                 380
   tta gat tca tgt gat gta gtt cct gga aat gga ata tta cgt ttg ttt        1200
   Leu Asp Ser Cys Asp Val Val Pro Gly Asn Gly Ile Leu Arg Leu Phe
   385                 390                 395                 400
   cac tct tac ttt aaa atg gtc aaa cat cta tcc tta ctg aag att aac        1248
   His Ser Tyr Phe Lys Met Val Lys His Leu Ser Leu Leu Lys Ile Asn
                   405                 410                 415
   agt aaa ttc gac tta ttg tta tgc agt tgt ttc cca tcg tta tcc aat        1296
   Ser Lys Phe Asp Leu Leu Leu Cys Ser Cys Phe Pro Ser Leu Ser Asn
               420                 425                 430
   cta aca att gat tgt aat agc aaa tgt ttc act aac gaa cag gta gtc        1344
   Leu Thr Ile Asp Cys Asn Ser Lys Cys Phe Thr Asn Glu Gln Val Val
           435                 440                 445
   ggc gaa tca tat tat ttc caa cag cga agt ttg gat aca gaa gat gat        1392
   Gly Glu Ser Tyr Tyr Phe Gln Gln Arg Ser Leu Asp Thr Glu Asp Asp
       450                 455                 460
   ttt gat gac tgc aat tcc atg aca gaa aca tta ttt gaa gcg cca tca        1440
   Phe Asp Asp Cys Asn Ser Met Thr Glu Thr Leu Phe Glu Ala Pro Ser
   465                 470                 475                 480
   gat tcg aag ata att act cct ccg ccg aca tct tca gtt gta ttg tcc        1488
   Asp Ser Lys Ile Ile Thr Pro Pro Pro Thr Ser Ser Val Val Leu Ser
                   485                 490                 495
   tta aat ctg aac tat att tct aga act aca gga aat gat gtt tca aat        1536
   Leu Asn Leu Asn Tyr Ile Ser Arg Thr Thr Gly Asn Asp Val Ser Asn
               500                 505                 510
   aac cca tca cca gac aat aac aaa aag ccc gcc atg tta act gcg gca        1584
   Asn Pro Ser Pro Asp Asn Asn Lys Lys Pro Ala Met Leu Thr Ala Ala
           515                 520                 525
   cag cta caa aat ttt caa cga caa aga atc cct gaa ttt cat tct ttc        1632
   Gln Leu Gln Asn Phe Gln Arg Gln Arg Ile Pro Glu Phe His Ser Phe
       530                 535                 540
   tac cat tat tac cgc tta ctt tgg gag aga ctt cca agt aaa aat att        1680
   Tyr His Tyr Tyr Arg Leu Leu Trp Glu Arg Leu Pro Ser Lys Asn Ile
   545                 550                 555                 560
   tct att aac gta atc aat atc ccc ttt acc aat gtg tat cct tta tcc        1728
   Ser Ile Asn Val Ile Asn Ile Pro Phe Thr Asn Val Tyr Pro Leu Ser
                   565                 570                 575
   cct cta tcg ttt tgg gaa cat cta gca aga aca att act agt gtt gat        1776
   Pro Leu Ser Phe Trp Glu His Leu Ala Arg Thr Ile Thr Ser Val Asp
               580                 585                 590
   gag aca gat gag gat gtt ggc gac gag aac gat caa gaa aca tta atc        1824
   Glu Thr Asp Glu Asp Val Gly Asp Glu Asn Asp Gln Glu Thr Leu Ile
           595                 600                 605
   ggt tac gaa aat aat tcc ata aga gat aac ata cca aat gct aat gca        1872
   Gly Tyr Glu Asn Asn Ser Ile Arg Asp Asn Ile Pro Asn Ala Asn Ala
       610                 615                 620
   gtt cca aat tta agt aca gta atg agc cct gaa tca gac att cac cac        1920
   Val Pro Asn Leu Ser Thr Val Met Ser Pro Glu Ser Asp Ile His His
   625                 630                 635                 640
   act tac tat tgg aac aac tct gta agg cgt tgc cta aga gat agt tta      1968
   Thr Tyr Tyr Trp Asn Asn Ser Val Arg Arg Cys Leu Arg Asp Ser Leu
                   645                 650                 655
   atc aaa ttg aag aac cga acc atc gaa tat aga gat tta gat gtg gag      2016
   Ile Lys Leu Lys Asn Arg Thr Ile Glu Tyr Arg Asp Leu Asp Val Glu
               660                 665                 670
   gag ttc tta caa aat gtg aca ttg gaa aat ttt ttc aat gat ttc caa      2064
   Glu Phe Leu Gln Asn Val Thr Leu Glu Asn Phe Phe Asn Asp Phe Gln
           675                 680                 685
   gat cca gag aat ttt aaa gat att cca aat att aac ctc tgg tgt ttc      2112
   Asp Pro Glu Asn Phe Lys Asp Ile Pro Asn Ile Asn Leu Trp Cys Phe
       690                 695                 700
   ctg aga aat ttg tca aaa ttc aaa gct gtc aaa ata aga atg ctg aga      2160
   Leu Arg Asn Leu Ser Lys Phe Lys Ala Val Lys Ile Arg Met Leu Arg
   705                 710                 715                 720
   cat ttt tca tta tgt aca cct aga acc aga tac gac tgg gaa cta tta      2208
   His Phe Ser Leu Cys Thr Pro Arg Thr Arg Tyr Asp Trp Glu Leu Leu
                   725                 730                 735
   ttg aag ccc gta ctg cgt gta aat gtt ccc att gaa gtc aga gat aag      2256
   Leu Lys Pro Val Leu Arg Val Asn Val Pro Ile Glu Val Arg Asp Lys
               740                 745                 750
   gac gga ttc gtt ctt tat tct tac ggg caa aaa taa                      2292
   Asp Gly Phe Val Leu Tyr Ser Tyr Gly Gln Lys
           755                 760
<210> 2
   <211> 763
   <212> PRT
   <213> Saccharomyces cerevisiae
<400> 2
   Met Lys Arg Leu Gln Leu Phe Gly Arg Ser Lys Tyr Phe Ser Leu Val
   1               5                   10                  15
   Ser Ser Ala Ala Lys Glu Glu Glu Glu Glu Glu Glu Gly Cys Ala Asp
               20                  25                  30
   Ala Lys Ser Leu Leu His Ser Thr Ser His Asp Ile Lys Ser Arg Ser
           35                  40                  45
   Leu Arg Phe Asn Asp Lys Ser Ser Leu Met Cys Leu Pro Thr Lys Val
       50                  55                  60
   Leu Leu Leu Ile Leu Arg Thr Leu Asp Phe Asn Thr Leu Val Thr Leu
   65                  70                  75                  80
   Cys Gln Val Asn Ser Arg Phe Tyr Asn Leu Ile Thr Asn Glu Phe Leu
                   85                  90                  95
   Phe Gln Asn Val Ile Leu Asp Ser Lys Leu Ser Leu Leu Lys Phe Asn
               100                 105                 110
   Ala Leu Ile His Ser Glu Phe His Thr Ser Asn Ile Val Thr His Ser
           115                 120                 125
   Gly Asp Cys Ser Thr Gln Ser Arg Ser Gln Asn Ala Arg Phe Leu Val
       130                 135                 140
   Arg Ser Ile Glu Phe Lys Asn Pro Gln Ser Gln Asp Ser Leu Leu Lys
   145                 150                 155                 160
   Tyr Ser Lys Phe Tyr Asn Lys Ser Gly Gln Asp Ser Ile Ile Ala Gly
                   165                 170                 175
   Ser Tyr Lys Leu Asp Ser Tyr Asp Lys Asp Val Lys Lys Leu Asn Asn
               180                 185                 190
   Ile Arg Leu Asn Asp Glu Thr Pro Ile Ile Thr Ser Glu Arg Ile Lys
           195                 200                 205
   Leu Leu Asp Lys Leu Glu Ser Asn Tyr Phe His Tyr Thr Tyr Ile Glu
       210                 215                 220
   Leu Met Leu Asp Ile Ile Asp Tyr Leu Pro Asn Leu Thr Arg Val Ile
   225                 230                 235                 240
   Leu Ser Asp Val Glu Pro Asn Phe Lys Ile Pro Leu Trp Tyr Ser Val
                   245                 250                 255
   Phe Asn Asp Gly Ser Arg Asp Phe Phe Lys Lys Ile Ile Lys Gly Gln
               260                 265                 270
   Gln Ser Ile Thr Asn Glu Asp Leu Arg Thr Phe Gln Leu Ser Lys Lys
           275                 280                 285
   Phe Val Lys Glu Tyr Glu Ser Lys Tyr Tyr Ser Leu Pro Arg Leu Lys
       290                 295                 300
   Ile Leu Glu Ile Lys Ala Asn Asn Lys Arg Gln Arg Thr Phe Asn Arg
   305                 310                 315                 320
   Gln Arg His His Gln Lys Leu Val Leu Arg Pro Ser Leu Phe Cys Cys
                   325                 330                 335
   Phe Gly Ile Ile Asn Glu Leu Lys Leu Glu Asn Val Thr Ile Asp Thr
               340                 345                 350
   Glu Ser Leu Asp Ile Pro Met Glu Phe Leu Pro Leu Phe Leu Lys Asn
           355                 360                 365
   Glu Asp Asn Glu Leu Tyr Ser Leu Gln Ser Pro Ile Thr Ala Leu Thr
       370                 375                 380
   Leu Asp Ser Cys Asp Val Val Pro Gly Asn Gly Ile Leu Arg Leu Phe
   385                 390                 395                 400
   His Ser Tyr Phe Lys Met Val Lys His Leu Ser Leu Leu Lys Ile Asn
                   405                 410                 415
   Ser Lys Phe Asp Leu Leu Leu Cys Ser Cys Phe Pro Ser Leu Ser Asn
               420                 425                 430
   Leu Thr Ile Asp Cys Asn Ser Lys Cys Phe Thr Asn Glu Gln Val Val
           435                 440                 445
   Gly Glu Ser Tyr Tyr Phe Gln Gln Arg Ser Leu Asp Thr Glu Asp Asp
       450                 455                 460
   Phe Asp Asp Cys Asn Ser Met Thr Glu Thr Leu Phe Glu Ala Pro Ser
   465                 470                 475                 480
   Asp Ser Lys Ile Ile Thr Pro Pro Pro Thr Ser Ser Val Val Leu Ser
                   485                 490                 495
   Leu Asn Leu Asn Tyr Ile Ser Arg Thr Thr Gly Asn Asp Val Ser Asn
               500                 505                 510
   Asn Pro Ser Pro Asp Asn Asn Lys Lys Pro Ala Met Leu Thr Ala Ala
           515                 520                 525
   Gln Leu Gln Asn Phe Gln Arg Gln Arg Ile Pro Glu Phe His Ser Phe
       530                 535                 540
   Tyr His Tyr Tyr Arg Leu Leu Trp Glu Arg Leu Pro Ser Lys Asn Ile
   545                 550                 555                 560
   Ser Ile Asn Val Ile Asn Ile Pro Phe Thr Asn Val Tyr Pro Leu Ser
                   565                 570                 575
   Pro Leu Ser Phe Trp Glu His Leu Ala Arg Thr Ile Thr Ser Val Asp
               580                 585                 590
   Glu Thr Asp Glu Asp Val Gly Asp Glu Asn Asp Gln Glu Thr Leu Ile
           595                 600                 605
   Gly Tyr Glu Asn Asn Ser Ile Arg Asp Asn Ile Pro Asn Ala Asn Ala
       610                 615                 620
   Val Pro Asn Leu Ser Thr Val Met Ser Pro Glu Ser Asp Ile His His
   625                 630                 635                 640
   Thr Tyr Tyr Trp Asn Asn Ser Val Arg Arg Cys Leu Arg Asp Ser Leu
                   645                 650                 655
   Ile Lys Leu Lys Asn Arg Thr Ile Glu Tyr Arg Asp Leu Asp Val Glu
               660                 665                 670
   Glu Phe Leu Gln Asn Val Thr Leu Glu Asn Phe Phe Asn Asp Phe Gln
           675                 680                 685
   Asp Pro Glu Asn Phe Lys Asp Ile Pro Asn Ile Asn Leu Trp Cys Phe
       690                 695                 700
   Leu Arg Asn Leu Ser Lys Phe Lys Ala Val Lys Ile Arg Met Leu Arg
   705                 710                 715                 720
   His Phe Ser Leu Cys Thr Pro Arg Thr Arg Tyr Asp Trp Glu Leu Leu
                   725                 730                 735
   Leu Lys Pro Val Leu Arg Val Asn Val Pro Ile Glu Val Arg Asp Lys
               740                 745                 750
   Asp Gly Phe Val Leu Tyr Ser Tyr Gly Gln Lys
           755                 760
<210> 3
   <211> 31
   <212> DNA
   <213> Saccharomyces cerevisiae
<400> 3
   atttctgggc aaaaasgtca aagcgtggtg t 31
<210> 4
   <211> 53
   <212> DNA
   <213> Saccharomyces cerevisiae
<400> 4
   ctagaaaatg taacgrtaga caccgaatcg ctagataytc caatggaatt ctt 53
<210> 5
   <211> 30
   <212> DNA
   <213> Saccharomyces cerevisiae
<400> 5
   atgttatgcc tgaggtatgt gtggtatcta 30
<210> 6
   <211> 30
   <212> DNA
   <213> Saccharomyces cerevisiae
<400> 6
   agtccactac aaaaagtcat ttatttttgc 30

## Description

La présente invention concerne le contrôle de la production de sulfites, d'hydrogène sulfureux et d'acétaldéhyde lors de la fermentation alcoolique par des levures.

Le dioxyde de soufre(SO₂) et ses différentes formes en équilibre en solution (HSO₃⁻, SO₃⁻⁻), collectivement désignés sous l'appellation de sulfites, sont utilisés comme additifs en oenologie, principalement pour améliorer la conservation des vins, du fait de ses propriétés anti-oxydantes et anti-bactériennes. Toutefois, une quantité trop importante de sulfites dans le vin peut entrainer des intolérances et des allergies chez certains consommateurs ; elle peut également être préjudiciable à ses qualités organoleptiques, vu qu'ils communiquent s'ils sont en excès, des sensations asséchantes. Des quantités trop élevées de sulfites à la fin de la fermentation alcoolique peuvent aussi être pénalisantes quand le vinificateur souhaite réaliser la fermentation malolactique. Les bactéries lactiques, responsables de cette fermentation sont inhibées par de faibles teneurs en sulfites, et un excès en retarde le déclenchement.
L'hydrogène sulfureux est également un métabolite formé par les levures en fermentation qui est préjudiciable pour la qualité de vins quand il est présent en excès en raison des goûts « d'oeuf pourri » ou de « réduit » qu'il communique.

Il est donc important de pouvoir optimiser la quantité de sulfites et d'hydrogène sulfureux dans les vins et en cours de vinification. Une difficulté majeure dans ce cadre provient du fait qu'une partie des sulfites et de l'hydrogène sulfureux présents dans le vin provient du métabolisme fermentaire des levures, où ils constituent des intermédiaires dans la synthèse des acides aminés soufrés. Le sulfate inorganique pénètre dans la cellule par l'intermédiaire d'une sulfate perméase. Il est activé en adénosylphosphosulfate (APS) par l'ATP-sulfurylase, puis l'APS est phosphorylé par l'adénosylphosphosulfate kinase pour produire du phosphoadénosylphosphosulfate (PAPS). Le PAPS est ensuite réduit en SO₂ par la PAPS réductase. Le SO₂ est réduit en H₂S par la sulfite réductase. L'homocystéine, qui est le précurseur des acides aminés soufrés, est synthétisée par réaction de l'H₂S avec l'O-acétylhomosérine, catalysée par l'O-acétylhomosérine sulfhydrylase.

La quantité de sulfites produits par les levures pendant la fermentation variant d'une souche de levure à l'autre, ceci complique le contrôle de la teneur globale en sulfites. Il en est de même pour l'hydrogène sulfureux dont la quantité formée dépend fortement de la souche de levure.

Un autre composé, dont la présence dans le vin au-delà de certaines quantités est considérée comme non-souhaitable est l'acétaldéhyde. L'acétaldéhyde à concentration trop élevée communique aux vins des notes dites « d'évent » qui sont considérées comme négatives. Il est produit par les levures au cours de la fermentation, et sa production apparait corrélée à la teneur en SO₂, et comme celle du SO₂, varie d'une souche de levure à l'autre.

Différentes approches ont été proposées pour obtenir des souches de levure produisant des quantités réduites de sulfites et/ou d'hydrogène sulfureux.

La Demande PCT WO2008/115759, et la Demande PCT WO2009/046485, ainsi que les publications de CORDENTE et al. (FEMS Yeast Res, 9, 446-59, 2009) et LINDERHOLM et al. (Appl Environ Microbiol, 76, 7699-707, 2010) décrivent différentes mutations dans les gènes MET5 ou MET10 (codant pour les 2 sous-unités catalytiques de la sulfite réductase), qui ont pour effet de diminuer la production d'hydrogène sulfureux. La Demande WO2009/030863 et la publication de MARULLO et al. (FEMS Yeast Res, 7, 1295-306, 2007), décrivent différents marqueurs associés à des caractères d'intérêt chez des levures oenologiques. L'un de ces marqueurs (YOL083w) situé sur le chromosome XV, est associé à une production réduite d'H₂S.

Les Inventeurs ont maintenant identifié des allèles de deux gènes impliqués dans le métabolisme du soufre chez *Saccharomyces,* comme étant associés à une production réduite de SO₂, d'acétaldéhyde, et dans le cas de l'un de ces gènes, de H₂S.

Le premier de ces gènes est le gène SKP2, situé sur le chromosome XIV (nt 49397 à 51688 dans la base « Saccharomyces genome database »). La séquence ADNc et la séquence polypeptidique correspondantes (pour la souche de référence de *Saccharomyces cerevisiae* S288C) sont disponibles dans la base de données GenBank sous les numéros d'accès respectifs NM_001183149.1 (GI:296147470) et NP_014088.1 (GI:6324018). SKP2 code pour une protéine de type F-box qui intervient dans la stabilité de différentes protéines du métabolisme du soufre et en particulier de l'adénosylphosphosulfate kinase responsable de la transformation de l'APS en PAPS. Il a été récemment montré (YOSHIDA et al., Yeast, 28, 109-21, 2011) que l'inactivation du gène SKP2 conduisait à une stabilisation de l'adénosylphosphosulfate kinase, et à une augmentation de la production d'H₂S et de SO₂.

Les Inventeurs ont identifié dans le gène SKP2 deux polymorphismes mononucléotidiques qui différencient la souche JN10 de la souche JN17: l'un en position 50 618 du chromosome XIV, où la souche JN10 possède un G et la souche JN17 possède un A, et l'autre en position 50 640 pb où la souche JN10 possède un C, tandis que la souche JN17 a un T. Ces polymorphismes se reflètent par le changement d'une valine pour JN10, en isoleucine pour JN17, à la position 350 de la protéine Skp2 (V350I), ainsi que d'une thréonine chez JN10 à la position 357 de Skp2, en isoleucine chez JN17 (T357I).

L'allèle du gène SKP2 présent chez la souche JN17 n'avait été identifié auparavant chez aucune autre souche de *Saccharomyces.* La séquence d'ADNc de cet allèle est indiquée dans la liste de séquences en annexe sous le numéro SEQ ID NO: 1, et la séquence polypeptidique déduite sous le numéro SEQ ID NO: 2.

Le second gène est le gène MET2, également situé sur le chromosome XIV (nt 117349 à 118809, coordonnées indiquées dans la base « Saccharomyces genome database » (http://www.yeastgenome.org) au 27/12/2011). La séquence ADNc et la séquence polypeptidique correspondantes (pour la souche de référence de *Saccharomyces cerevisiae* S288C) sont disponibles dans la base de données GenBank sous les numéros d'accès respectifs NM_001183115.1 (GI:296147504) et NP_014122.1 (GI:6324052). MET2 code pour l'homosérine-O-acétyl transférase qui catalyse la conversion de l'homosérine en O-acétyl homosérine qui est ensuite condensée avec H₂S pour former l'homocystéine. Il a été montré (HANSEN & KIELLAND-BRANDT, J Biotechnol, 50, 75-87, 1996) que l'inactivation du gène MET2 chez *Saccharomyces* entrainait une augmentation de la production de sulfites et d'hydrogène sulfureux.

Les Inventeurs ont identifié en position 118 249 du chromosome XIV, un polymorphisme mononucléotidique qui différencie les gènes MET2 de deux souches de *Saccharomyces cerevisiae,* l'une (souche JN10) forte productrice de SO₂, H₂S et d'acétaldéhyde dans certaines conditions de fermentation, et l'autre (souche JN17) faible productrice de ces mêmes composés. La souche JN10 possède un C tandis que la souche JN17 possède (comme la souche de référence S288C) un G, ce qui entraîne un changement d'acide aminé et la conversion d'une arginine chez la souche JN10 en glycine chez la souche JN17 en position 301 de la protéine Met2 (R301G).

La présente invention a pour objet une méthode d'obtention d'une souche de levure du genre *Saccharomyces* produisant une quantité de SO₂, d'hydrogène sulfureux et d'acétaldéhyde plus faible que celle produite par la souche mère dont elle est issue, ladite méthode étant caractérisée en ce qu'elle comprend :
- la sélection d'une souche mère contenant un allèle du gène SKP2, ci-après dénommé SKP2^{(350/357)X}, codant pour une protéine Skp2 dans laquelle l'acide aminé en position 350 et/ou l'acide aminé en position 357 est (sont) autre(s) qu'une (des) isoleucine(s);
- l'introduction dans ladite souche mère d'un allèle du gène SKP2, ci-après dénommé SKP2^{(350/357)I}, codant pour une protéine Skp2 dans laquelle l'acide aminé en position 350 et l'acide aminé en position 357 sont des isoleucines.
Par exemple, si la souche mère contient un allèle SKP2^{(350/357)X} et un allèle MET2^{301G}, on pourra y introduire un allèle SKP2^{(350/357)I}. Inversement, si la souche mère contient un allèle SKP2^{(350/357)I} et un allèle MET2^{301X}, on pourra y introduire un allèle MET2^{301G}. Si la souche mère contient un allèle SKP2^{(350/357)X} et un allèle MET2^{301X}, on peut choisir d'y introduire soit un allèle SKP2^{(350/357)I}, soit un allèle MET2^{301G}. De préférence, on choisira d'y introduire à la fois un allèle SKP2^{(350/357)I} et un allèle MET2^{301G}.

Dans le cadre de l'exposé de la présente invention, la dénomination « allèle SKP2^{(350/357)I} » englobe: un allèle (plus spécifiquement dénommé allèle SKP2 ^{350I/357X}) codant pour une protéine Skp2 dans laquelle l'acide aminé en position 350 est une isoleucine et l'acide aminé en position 357 est autre qu'une isoleucine ; un allèle (plus spécifiquement dénommé allèle SKP2 ^{350X/357I}) codant pour une protéine Skp2 dans laquelle l'acide aminé en position 350 est autre qu'une isoleucine ; un allèle (plus spécifiquement dénommé allèle SKP2 ^{350I/357I}) dans lequel l'acide aminé en position 350 et l'acide aminé en position 357 sont tous deux des isoleucines, ce dernier allèle étant particulièrement préféré.

Selon un mode de mise en oeuvre préféré de la présente invention, ladite souche mère contient un allèle du gène SKP2, ci-après dénommé SKP2^{350V/357T}, codant pour une protéine Skp2 dans laquelle l'acide aminé en position 350 est une valine et/ou l'acide aminé en position 357 est une thréonine. Avantageusement ladite souche de levure appartient à l'espèce *Saccharomyces cerevisiae.*

L'allèle SKP2^{(350/357)I} peut être introduit dans la souche mère par différentes méthodes, bien connues en elles-mêmes de l'homme du métier. Il peut être introduit par exemple par croisement avec une souche possédant l'allèle SKP2^{(350/357)I} recherché, et sélection parmi les descendants de ce croisement, de ceux auxquels ledit allèle a été transmis.

L'allèle SKP2^{(350/357)I} peut également être introduit en remplacement de l'allèle initial (SKP2^{(350/357)X}) ou en supplément de celui-ci, en utilisant des techniques classiques de génie génétique (cf. par exemple AMBERG et al., Methods in yeast genetics: a Cold Spring Harbor Laboratory course manual, Cold Spring Harbor Laboratory Press, 2005).

Si la méthode conforme à l'invention est mise en oeuvre à partir d'une souche mère haploide portant l'allèle SKP2^{(350/357)X}, l'introduction dans la dite souche d'une copie de l'allèle SKP2^{(350/357)I} par croisement produit une souche hétérozygote SKP2^{(350/357)X}/SKP2^{(350/357)I}, produisant une quantité de sulfites, d'hydrogène sulfureux et d'acétaldéhyde plus faible que celle produite par la souche mère dont elle est issue. Il est aussi possible d'obtenir des descendants haploides de cette souche qui possèdent l'allèle SKP2^{(350/357)I} et produisent donc de faibles quantités de sulfites, d'hydrogène sulfureux et d'acétaldéhyde. Par des séries de croisements en retour entre des descendants possédant l'allèle SKP2^{(350/357)I} et la souche mère, il est ainsi possible d'obtenir une souche avec un génome proche de celui de la souche mère, ayant acquis l'allèle SKP2^{(350/357)I} et produisant de faibles quantités de sulfites, d'hydrogène sulfureux et d'acétaldéhyde.

La présente invention a également pour objet un polynucléotide isolé codant pour la protéine Skp2 de séquence SEQ ID NO: 2, qui correspond à l'allèle SKP2^{350I/357I}.

Selon un mode de réalisation préféré de la présente invention, ce polynucléotide est défini par la séquence SEQ ID NO: 1.

Ce polynucléotide peut être utilisé pour dans le cadre de la méthode conforme à l'invention décrite ci-dessus, pour introduire dans une souche de levure l'allèle SKP2^{350I/357I}

La présente invention a également pour objet un vecteur d'acide nucléique contenant un polynucléotide de séquence SEQ ID NO: 1, ou un fragment de celui-ci contenant au moins la région 1045-1075 de SEQ ID NO: 1.

Ledit vecteur peut être tout type de vecteur utilisable chez la levure, notamment chez Saccharomyces. De tels vecteurs sont bien connus en eux-mêmes. On peut utiliser par exemple des vecteurs réplicatifs extrachromosomiques, tels que les vecteurs Yep ou les vecteurs Yrp. On peut également utiliser des vecteurs intégratifs tels que les vecteurs Yip.

Dans le cas d'un vecteur intégratif, le polynucléotide de séquence SEQ ID NO: 1, ou ledit fragment est flanqué en amont et en aval de séquences d'au moins 20pb, de préférence de 40 à 60 pb, homologues à celles flanquant le gène SKP2 ou la région 1045-1075 dudit gène chez la souche dans laquelle on souhaite introduire l'allèle SKP2^{350I/357I}.

Le fragment d'ADN contenant la séquence SEQ ID NO: 1, ou au moins la région 1045-1075 de SEQ ID NO: 1, sera associé éventuellement à un gène marqueur (gène codant pour un protéine conférant la résistance à un inhibiteur ou gène permettant de complémenter une mutation responsable d'une auxotrophie de la souche réceptrice) facilitant la sélection des clones ayant acquis le fragment par transformation.

La présente invention a également pour objet une méthode pour évaluer la capacité d'une souche de *Saccharomyces,* de préférence de *Saccharomyces cerevisiae* à produire du SO₂, de l'hydrogène sulfureux et de l'acétaldéhyde, caractérisée en ce qu'elle comprend :
- le génotypage de ladite souche pour le gène SKP2, et la détection de la présence d'un allèle SKP2^{(350/357)X} et notamment de l'allèle SKP2^{350V/357T}, et/ou d'un allèle SKP2^{(350/357)I}, et notamment de l'allèle SKP2^{350I/357I}; et/ou
- le génotypage de ladite souche pour le gène SKP2, et la détection de la présence d'un allèle SKP2^{(350/357)X} et notamment de l'allèle SKP2^{350V/357T}, et/ou d'un allèle SKP2^{(350/357)I}, et notamment de l'allèle SKP2^{350I/357I}.

La présente demande décrit également des réactifs permettant la mise en oeuvre de la méthode de génotypage conforme à l'invention.

Ces réactifs comprennent notamment :
- des sondes oligonucléotidiques allèle-spécifiques, permettant de différencier l'allèle SKP2^{350V/357T} d'un allèle SKP2^{(350/357)I}, et notamment de l'allèle SKP2^{350I/357I} ou de différencier l'allèle MET2^{301R} de l'allèle MET2^{301G}, en s'hybridant sélectivement avec l'un ou l'autre des allèles à différencier.
- des amorces spécifiques, permettant de différencier l'allèle SKP2^{350V/357T} d'un allèle SKP2^{(350/357)I}, et notamment de l'allèle SKP2^{350I/357I}, ou de différencier l'allèle MET2^{301R} de l'allèle MET2^{301G}, ainsi que des kits d'amorces contenant au moins une amorce spécifique conforme à l'invention. Généralement, ces kits d'amorces comprennent une amorce spécifique pour chaque allèle à détecter, et une amorce commune, capable de s'hybrider, dans les mêmes conditions d'amplification, avec tous les allèles du gène concerné.

Des sondes permettant de différencier l'allèle SKP2^{350V/357T} d'un allèle SKP2^{(350/357)I}, et notamment de l'allèle SKP2^{350I/357I} peuvent par exemple être constituées par des fragments de 15 à 30 pb de la séquence : CTAGAAAATGTAACGRTAGACACCGAATCGCTAGATAYTCCAATGGAATTCT T (SEQ ID NO:4, où A, T, C, G, R, et Y ont leur signification usuelle en code IUPAC) lesdits fragments contenant au moins le locus du polymorphisme G/A, ou au moins le locus du polymorphisme C/T, et le cas échéant les 2 loci polymorphes de ladite séquence, ou par leurs complémentaires.

Les sondes dans lesquelles R=G, ainsi que les sondes dans lesquelles Y=C peuvent s'hybrider sélectivement avec l'allèle SKP2^{350V/357T} tandis que les sondes dans lesquelles R=A et celles dans lesquelles Y= T peuvent s'hybrider sélectivement avec un allèle SKP2^{(350/357)I}, et notamment l'allèle SKP2^{350I/357I}.

Des sondes permettant de différencier l'allèle MET2^{301R} de l'allèle MET2^{301G} peuvent par exemple être constituées par des fragments de 15 à 30 pb de la séquence : ATTTCTGGGCAAAAASGTCAAAGCGTGGTGT (SEQ ID NO: 3, où A, T, C, G, et S ont leur signification usuelle en code IUPAC), lesdits fragments contenant le locus du polymorphisme C/G de ladite séquence, ou par leurs complémentaires. Les sondes dans lesquelles S=C peuvent s'hybrider sélectivement avec l'allèle MET2^{301R} tandis que les sondes dans lesquelles S=G peuvent s'hybrider sélectivement avec l'allèle MET2^{301G}.

Des amorces spécifiques permettant de différencier SKP2^{350V} de SKP2^{350I} peuvent par exemple être constituées par des fragments de 15 à 30 pb de la séquence SEQ ID NO: 4 contenant au moins le locus du polymorphisme G/A ou son complémentaire. Les amorces dans lesquelles R=G peuvent être utilisées pour l'amplification sélective de SKP2^{350V} tandis que les amorces dans lesquelles R=A peuvent être utilisées pour l'amplification sélective de SKP2^{350I} Des amorces spécifiques conformes à l'invention permettant de différencier SKP2^{357T} de SKP2^{357I} peuvent par exemple être constituées par des fragments de 15 à 30 pb de la séquence SEQ ID NO: 4 contenant au moins le locus du polymorphisme C/T, ou leurs complémentaires.

Les amorces dans lesquelles Y=C peuvent être utilisées pour l'amplification sélective de SKP2^{357T} et celles dans lesquelles Y= T peuvent être utilisées pour l'amplification sélective de SKP2^{357I}. Un kit d'amorces permettant de différencier l'allèle SKP2^{350V/357T} d'un allèle SKP2^{(350/357)I}, comprend une paire d'amorces spécifiques permettant de différencier SKP2^{350V} de SKP2^{350I}, et une paire d'amorces spécifiques permettant de différencier SKP2^{357T} de SKP2^{357I}.

Des amorces spécifiques permettant de différencier l'allèle MET2^{301R} de l'allèle MET2^{301G} peuvent par exemple être constituées par des fragments de 15 à 30 pb de la séquence SEQ ID NO: 3 contenant au moins le locus du polymorphisme C/G de ladite séquence, ou par leurs complémentaires. Les amorces dans lesquelles S=C peuvent être utilisées pour l'amplification sélective de l'allèle MET2^{301R} tandis que les amorces dans lesquelles S=G peuvent être utilisées pour l'amplification sélective de l'allèle MET2^{301G}.

Des amorces communes pouvant être utilisées en association avec les amorces spécifiques permettant de différencier l'allèle MET2^{301R} de l'allèle MET2^{301G} dans les kits d'amorces conformes à l'invention peuvent par exemple être constituées par des fragments de 15 à 30 pb de la séquence suivante : ATGTTATGCCTGAGGTATGTGTGGTATCTA (SEQ ID NO: 5, où A, T, C, et G ont leur signification usuelle en code IUPAC), ou par leurs complémentaires.

Des amorces communes pouvant être utilisées en association avec les amorces spécifiques permettant de différencier SKP2^{350V} de SKP2^{350I} et/ou avec les amorces spécifiques permettant de différencier SKP2^{357T} de SKP2^{357I} dans les kits d'amorces conformes à l'invention peuvent par exemple être constituées par des fragments de 15 à 30 pb de la séquence suivante : AGTCCACTACAAAAAGTCATTTATTTTTGC (SEQ ID NO: 6, où A, T, C, et G ont leur signification usuelle en code IUPAC), ou par leurs complémentaires.

La présente invention sera mieux comprise à l'aide du complément de description qui va suivre, qui se réfère à des exemples non-limitatifs illustrant les effets des allèles des gènes MET2 et SKP2 sur la production de SO₂, d'hydrogène sulfureux, et d'acétaldéhyde.

### EXEMPLE 1 : EFFET DES ALLÈLES DU GÈNE MET2 SUR LA PRODUCTION DE SO₂, D'HYDROGÈNE SULFUREUX, ET D'ACÉTALDÉHYDE.

La souche JN10 de *Saccharomyces cerevisiae* (forte productrice de SO₂, H₂S et acétaldéhyde), possède un allèle du gène MET2 qui code pour une protéine Met2 dans laquelle l'acide aminé en position 301 est une arginine, alors que la souche JN17 (faible productrice de ces mêmes composés) possède un allèle du gène MET2 codant pour une protéine Met2 dans laquelle l'acide aminé en position 301 est une glycine.

L'impact du remplacement de l'allèle MET2 de JN10 (MET2^{JN10}) par celui de JN17 (MET2^{JN17}), ou inversement, celui du remplacement de l'allèle MET2 de JN17 par celui de JN10 ont été évalués.

Dans un premier temps l'allèle initial MET2^{JN10} ou MET2^{JN17} a été délété et remplacé par une cassette contenant un gène de résistance à la généticine *(KANMX4),* selon la méthode décrite par WACH et al. (Yeast, 10, 1793-808, 1994). Les cellules transformées sont sélectionnées sur la base de leur résistance à l'antibiotique, et de leur auxotrophie pour la méthionine.

L'allèle MET2^{JN17} amplifié à partir de l'ADN génomique de la souche JN17 a été ensuite introduit en remplacement de la cassette de résistance à la généticine dans la souche JN10, et réciproquement, l'allèle MET2^{JN10} amplifié à partir de l'ADN génomique de la souche JN10 a été introduit en remplacement de la cassette de résistance à la généticine dans la souche JN17. Les souches transformées sont sélectionnées sur la base de la restauration de leur prototrophie pour la méthionine.

Les impacts du changement allélique sur la formation de SO₂, de H₂S, et d'acétaldéhyde ont été évalués lors de fermentations alcooliques en conditions oenologiques.

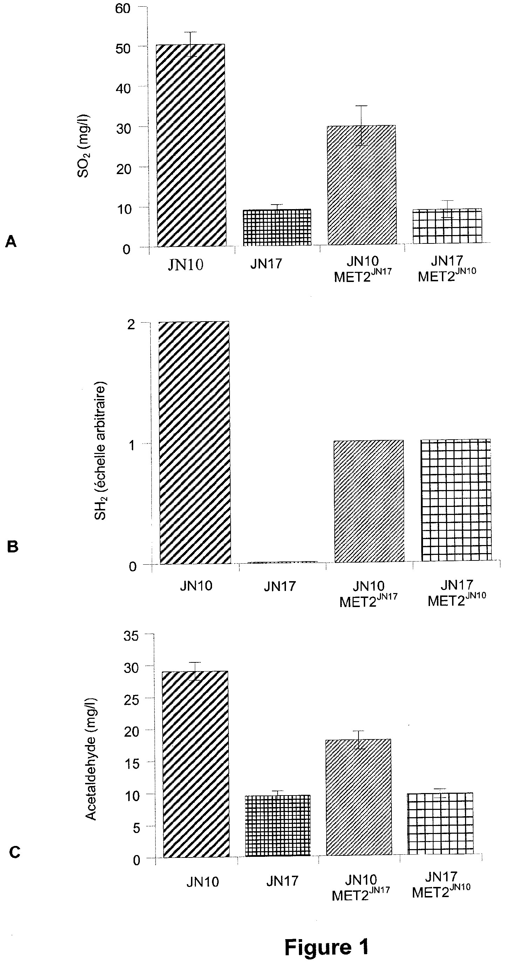

Les résultats sont représentés sur la Figure 1. A : production de SO₂ ; B : production de H₂S ; C : production d'acétaldéhyde.

Le remplacement de l'allèle MET2^{JN10} par MET2^{JN17} dans la souche JN10 (souche JN10-MET2^{JN17}) entraîne une réduction de la concentration en SO₂ formé d'environ 40 %. De même, la production de H₂S est réduite de manière significative, 1 sur une échelle allant de 0 à 2. Le niveau d'acétaldéhyde est également diminué de près de 40%. Le remplacement allélique inverse (allèle MET2^{JN10} dans le fonds génétique de la souche JN17 : souche JN17 MET2^{JN10}) n'a pas d'impact sur la production de SO₂, ni sur celle d'acétaldéhyde ; en revanche, on observe une augmentation de la production de H₂S par rapport à la souche parentale JN17.

### EXEMPLE 2 : EFFET DES ALLÈLES DU GÈNE SKP2 SUR LA PRODUCTION DE SO₂, D'ACÉTALDÉHYDE, ET D'HYDROGÈNE SULFUREUX.

L'allèle du gène SKP2 présent chez la souche JN10 de *Saccharomyces cerevisiae* (SKP2^{JN10}) code pour une protéine Skp2 dans laquelle l'acide aminé en position 350 est une valine et l'acide aminé en position 357 est une thréonine, alors que l'allèle présent chez la souche JN17 (SKP2^{JN17}) code pour une protéine Skp2 dans laquelle les acides aminés en positions 350 et 357 sont des isoleucines.

L'impact de la forme allélique du gène SKP2 (SKP2^{JN10} ou SKP2^{JN17}) a été évalué par construction d'hémizygotes. Le remplacement allélique était en effet une méthode plus délicate à mettre en oeuvre que dans le cas du gène MET2 car l'inactivation du gène SKP2 ne conduit qu'à un retard de croissance sur milieu minimum (YOSHIDA et al., 2011, précité), phénotype qui contrairement à l'auxotrophie à la méthionine observée dans le cas du gène MET2, n'est pas aisément utilisable comme marqueur de sélection.

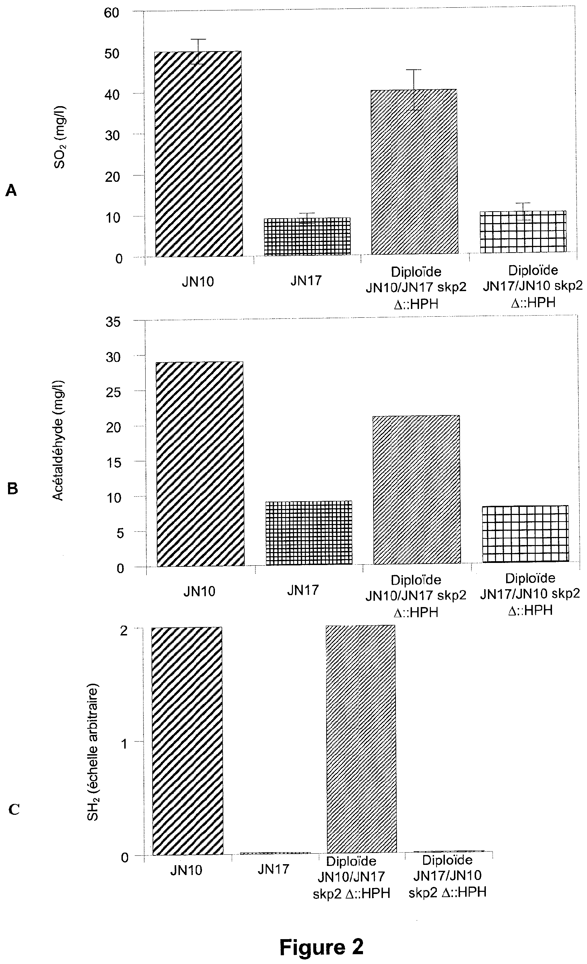

Dans un premier temps, le gène SKP2 a été inactivé chez chacune des souches parentales JN10 et JN17, par insertion de la cassette HPH qui confère la résistance à l'hygromycine B, pour obtenir respectivement les souches JN10skp2Δ::HPH et la souche JN17skp2Δ::HPH. La souche JN10skp2Δ::HPH a ensuite été croisée avec la souche JN17, et la souche JN17skp2Δ::HPH avec la souche JN10, pour obtenir respectivement les souches diploïdes JN17/JN10skp2Δ::HPH et JN10/JN17 skp2A::HPH, qui ne possèdent qu'un seul allèle fonctionnel de SKP2 (respectivement l'allèle SKP2^{JN17} et l'allèle SKP2^{JN10}). La production de sulfites, d'acétaldéhyde et d'hydrogène sulfureux par ces souches hémizygotes pour SKP2 a été évaluée en conditions de fermentations alcooliques oenologiques. Les résultats sont illustrés par la Figure 2. A : production de SO₂ ; B : production d'acétaldéhyde ; C : production d'hydrogène sulfureux.

On constate que la production de SO₂ est plus faible chez l'hémizygote qui possède l'allèle SKP2^{JN17} que chez celui qui possède l'allèle SKP2^{JN10}. De même, la teneur en acétaldéhyde est plus faible quand l'allèle SKP2^{JN17} est actif que lorsque c'est celui issu de la souche JN10. Enfin la teneur en hydrogène sulfureux est plus faible quand l'allèle SKP2^{JN17} est actif que lorsque c'est celui issu de la souche JN10. L'allèle SKP2^{JN17} conduit donc à une réduction des teneurs en SO₂, en acétaldéhyde et en hydrogène sulfureux.

### EXEMPLE 3 : EFFET COMBINÉ DES ALLÈLES DES GÈNES MET2 ET SKP2 SUR LA PRODUCTION DE SO₂ ET D'HYDROGÈNE SULFUREUX

L'impact d'une combinaison des deux formes alléliques SKP2^{JN17} et MET2^{JN17} a été évalué grâce à la construction de souches quasi-isogéniques possédant plus de 93% du génome de la souche JN10 suite à des cycles de rétro-croisements. Les rétro-croisements consistent en une série de croisements successifs avec la même souche, (ici JN10). La souche JN17 est tout d'abord hybridée avec la souche JN10. L'hybride obtenu, possédant 50% du génome de la souche JN10 et 50 % du génome de la souche JN17 et présentant le génotype suivant : SKP2^{JN17}/SKP2^{JN10} et MET2^{JN17}/MET2^{JN10}, est induit à sporuler. Après sporulation les spores haploïdes possédant le génotype suivant : SKP2^{JN17} et MET2^{JN17} sont sélectionnées par des PCR allèle-spécifique pour ces deux gènes. Ces spores sont alors croisées à nouveau avec la souche JN10. Un nouvel hybride est obtenu, possédant 75% du génome de la souche JN10 et 25% du génome de la souche JN17 et présentant le génotype suivant : SKP2^{JN17}/SKP2^{JN10} et MET2^{JN17}/MET2^{JN10}, cet hybride est à son tour induit à sporuler. Les asques sont disséqués et une spore possédant le génotype suivant: SKP2^{JN17} et MET2^{JN17} est sélectionnée. Les cycles de croisement/sporulation/sélection d'une spore sont poursuivis jusqu'à obtenir des dérivés présentant un pourcentage du génome de la souche JN10 très élevé, ici 93.25%.

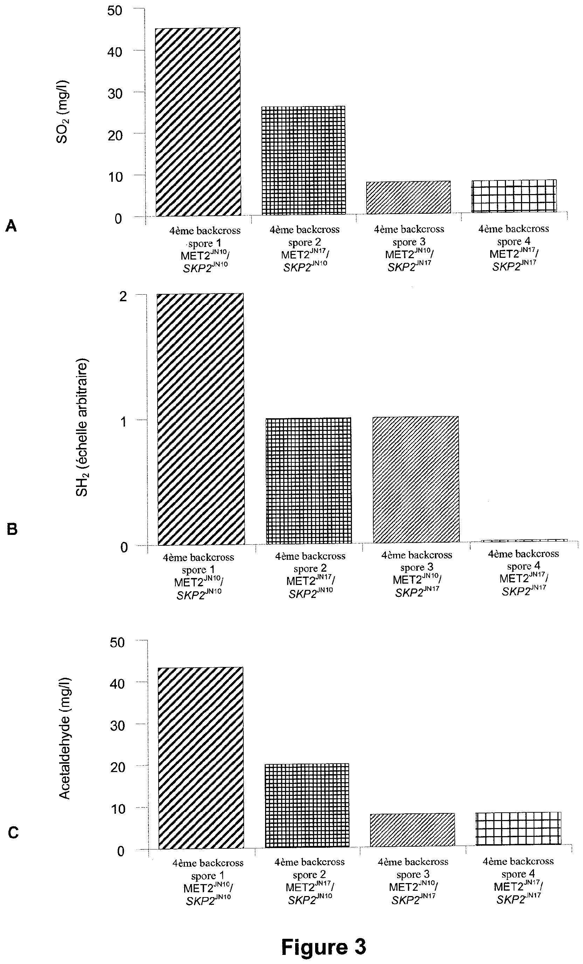

Par sporulation des clones diploïdes obtenus lors du dernier cycle, on obtient des dérivés haploïdes (Spores 4^{ème} backcross 1 à 4) présentant les combinaisons d'allèles suivantes : SKP2^{JN17}/MET2^{JN17} ; SKP2^{JN10}/MET2^{JN17} ; SKP2^{JN17}/MET2^{JN10} ; SKP2^{JN10}/MET2^{JN10} dans des fonds génétiques quasi-identiques. La production de SO₂, d'H₂S, et d'acétaldéhyde de ces différents dérivés a été évaluée en conditions de fermentation alcoolique oenologique. Les résultats sont illustrés par le Tableau I ci-dessous, et par la Figure 3.

**Tableau I**

| Allele SKP2 | Allele MET2 | SO2 (mg/l) | H2S | Acétaldéhyde (mg/L) |
|---|---|---|---|---|
| JN10 | JN10 | 46 | 2 | 43 |
| JN10 | JN17 | 28 | 1 | 20 |
| JN17 | JN10 | 5 | 1 | 6 |
| JN17 | JN17 | 5 | 0 | 6 |

| | | | | |
|---|---|---|---|---|
| Echelle H2S : 0 = production non détectée, 1 = production moyenne, 2 = production torte | | | | |

On constate que la production de SO₂ d'un dérivé possédant les deux allèles SKP2^{JN10}/MET2^{JN10} est identique à celle de la souche initiale JN10, alors qu'un dérivé possédant une combinaison d'allèles de type SKP2^{JN10}/MET2^{JN17} produit des quantités de SO₂ intermédiaires. Par ailleurs, des dérivés possédant soit la combinaison d'allèles SKP2^{JN17}/MET2^{JN10} soit les deux allèles de la souche JN17, SKP2^{JN17}/MET2^{JN17}, produisent tous deux des quantités de SO₂ très faibles et identiques à celles de la souche initiale JN17.

L'effet des différentes combinaisons d'allèles sur la production d'acétaldéhyde est identique à celui observé sur la production de SO2.

D'autre part, les dérivés possédant les deux allèles de la souche JN10 produisent de fortes quantités d'H2S, identiques à la souche parentale JN10, tandis que les dérivés possédant un des deux allèles de la souche JN17, possédant donc les génotypes suivants : SKP2^{JN17}/MET2^{JN10} ou SKP2^{JN10}/MET2^{JN17}, produisent de l'H₂S en quantités intermédiaires et seul le dérivé possédant les deux allèles SKP2^{JN17}/MET2^{JN17} ne produit pas d'H2S détectable, de la même manière que la souche parentale JN17.

## Revendications

1. Méthode d'obtention d'une souche de levure du genre Saccharomyces produisant une quantité de SO2, d'hydrogène sulfureux et d'acétaldéhyde plus faible que celle produite par la souche mère dont elle est issue, ladite méthode étant **caractérisée en ce qu'**elle comprend :
- la sélection d'une souche mère contenant un allèle du gène SKP2, ci-après dénommé SKP2^{(350/357)x} codant pour une protéine Skp2 dans laquelle l'acide aminé en position 350 et/ou l'acide aminé en position 357 est (sont) autre(s) qu'une (des) isoleucine(s) ;
- l'introduction dans ladite souche mère d'un allèle du gène SKP2, ci-après dénommé SKP2^{(350/357)I} codant pour une protéine Skp2 dans laquelle l'acide aminé en position 350 et l'acide aminé en position 357 sont des isoleucines.

2. Méthode selon la revendication 1 , **caractérisée en ce que** ladite souche de levure appartient à l'espèce Saccharomyces cerevisiae.

3. Polynucléotide isolé codant pour la protéine Skp2 définie par la séquence SEQ ID NO: 2.

4. Polynucléotide selon la revendication 3 , défini par la séquence SEQ ID NO: 1.

5. Vecteur d'acide nucléique contenant un polynucléotide selon une quelconque des revendications 3 ou 4.

6. Méthode pour évaluer la capacité d'une souche de Saccharomyces à produire du SO2, de l'hydrogène sulfureux, et de l'acétaldéhyde, **caractérisée en ce qu'**elle comprend :
- le génotypage de ladite souche pour le gène SKP2, et la détection de la présence d'un allèle SKP2 (350/357)^{x} et/ou d'un allèle SKP2^{(350/357)I}.

## Patentansprüche

1. Verfahren zum Erhalt eines Hefestamms der Sorte Saccharomyces, der eine Menge S02, Schwefelwasserstoff und Acetaldehyd produziert, die geringer ist als diejenige, die vom Mutterstamm produziert wird, von dem er abstammt, wobei das Verfahren **dadurch gekennzeichnet ist, dass** es umfasst :
- die Auswahl eines Mutterstamms, welcher ein Allel des Gens SKP2 enthält, nachfolgend bezeichnet als SKP2^{(350/357)X}, codierend für ein Protein Skp2, in welchem die Aminosäure an Position 350 und/oder die Aminosäure an Position 357 (eine) andere sind (ist) als ein(es) (der) Isoleucin(e),
- Einsetzen in den Mutterstamm eines Allels des Gens SKP2, nachfolgend bezeichnet als SKP2^{(350/357)I}, codierend für ein Protein Skp2, in welchem die Aminosäure an Position 350 und/oder die Aminosäure an Position 357 Isoleucin(e) ist/sind.

2. Verfahren nach Anspruch 1, **dadurch gekennzeichnet, dass** der Hefestamm zur Art Saccharomyces cerevisiae gehört.

3. Isoliertes Polynukleotid, codierend für das Protein Skp2, das von der Sequenz SEQ ID NO: 2 definiert ist.

4. Polynukleotid nach Anspruch 3, das von der Sequenz SEQ ID NO: 1 definiert ist.

5. Nukleinsäurevektor, enthaltend ein Polynukleotid nach einem der Ansprüche 3 oder 4.

6. Verfahren zur Beurteilung der Fähigkeit eines Stamms von Saccharomyces, S02, Schwefelwasserstoff und Acetaldehyd zu produzieren, **dadurch gekennzeichnet, dass** es umfasst:
- die Genotypisierung des Stamms für das Gen SKP2 und die Detektion der Abwesenheit eines Allels SKP2^{(350/357)X} und/oder eines Allels SKP2^{(350/357)I}.

## Claims

1. Method for obtaining a yeast strain of the genus *Saccharomyces* producing a lower amount of SO₂, hydrogen sulphide and acetaldehyde than that produced by the parent strain from which it is derived, said method being **characterized in that** it comprises:
- selecting a parent strain containing an allele of the SKP2 gene, hereafter designated SKP2^{(350/357)x,} encoding a Skp2 protein wherein the amino acid at position 350 and/or the amino acid at position 357 is (are) other than isoleucine(s);
- inserting in said parent strain an allele of the SKP2 gene, hereafter designated SKP2^{350/357)I}, encoding a Skp2 protein wherein the amino acid at position 350 and the amino acid at position 357 are isoleucines.

2. The method according to claim 1, **characterized in that** said yeast strain belongs to the species *Saccharomyces cerevisiae.*

3. Isolated polynucleotide coding for the Skp2 protein defined by sequence SEQ ID NO:2.

4. The polynucleotide according to claim 3, defined by sequence SEQ ID NO:1.

5. Nucleic acid vector containing a polynucleotide according to any of claims 3 or 4.

6. Method for evaluating the capacity of a strain of *Saccharomyces* to produce SO₂, hydrogen sulphide and acetaldehyde, **characterized in that** it comprises:
- genotyping said strain for the SKP2 gene, and detection of the presence of an allele SKP2(350/357)^{x} and/or an allele SKP2^{(350/357)I}.
